# EUROPEAN PATENT SPECIFICATION

(11) **EP 0 649 418 B1**
(45) Date of publication and mention of the grant of the patent: **28.10.1998**
(21) Application number: 93913203.1
(22) Date of filing: 27.05.1993
(51) Int. Cl.: C07D 277/68, A61K 31/425

(54) **7-(2-AMINOETHYL)-BENZOTHIAZOLONES**
7-(2-AMINOETHYL)-BENZOTHIAZOLONE
7-(2-AMINOETHYL)- BENZOTHIAZOLONES

(30) Priority: 27.05.1992 GB 9211172
(43) Date of publication of application: 26.04.1995
(73) Proprietor: Astra Pharmaceuticals Limited, Kings Langley, Herts WD4 8DH (GB)
(72) Inventor: BONNERT, Roger Victor, Loughborough, Leicestershire LE12 5SE (GB); BROWN, Roger Charles, Loughborough, Leicestershire LE11 0QN (GB); CHESHIRE, David Ranulf, Nottinghamshire NG9 4DA (GB); INCE, Francis, Loughborough, Leicestershire LE11 3SQ (GB)
(74) Representative: As Sivborg, Susanne Birgitta
(86) International application number: GB9301095
(87) International publication number: WO9324473

(56) References cited:
- EP-A- 0 174 811
- WO-A-92/08708

## Description

This invention relates to novel compounds, processes for their preparation, pharmaceutical compositions containing them and methods of treatment involving their use.

International Patent Application No. WO 92/08708 (published after the priority date of this application) discloses a number of biologically active amines and their activity as β₂-adrenoreceptor agonists and dopamine DA₂-agonists.

We have now found a group of 7-(2-aminoethyl)-benzothiazolone derivatives which exhibit significant advantages over those known from the prior art.

According to the invention there are provided compounds of formula I, 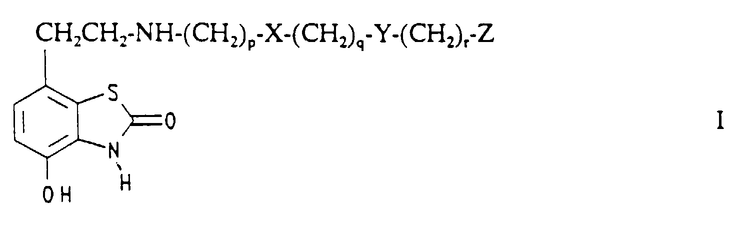 wherein
X and Y independently represent -S(O)ₙ- or -O-,
n represents 0, 1 or 2,
p, q and r independently represent 2 or 3,
Z represents phenyl optionally substituted by halogen, hydroxyl, methoxy, nitro or amino, or represents a pyridyl or thienyl group,
and pharmaceutically acceptable derivatives thereof.

According to the invention we also provide a process for the production of a compound of formula I, or a pharmaceutically acceptable salts thereof, which comprises
a) alkylation of a compound of formula II, 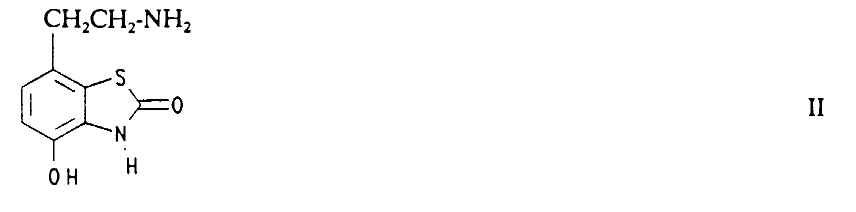 with an alkylating agent of formula III,

   L-(CH₂)ₚ-X-(CH₂)_{q}-Y-(CH₂)ᵣ-Z III

   in which p, q, r, X, Y and Z are as defined above and L represents a leaving group,
b) alkylation of a compound of formula II, as defined above, with a compound of formula IV,

   O=CH-(CH₂)ₚ₋₁-X-(CH₂)_{q}-Y-(CH₂)ᵣ-Z IV

   in which p, q, r, X, Y and Z are as defined above,
   in the presence of a reducing agent,
c) selective reduction of a compound of formula V, 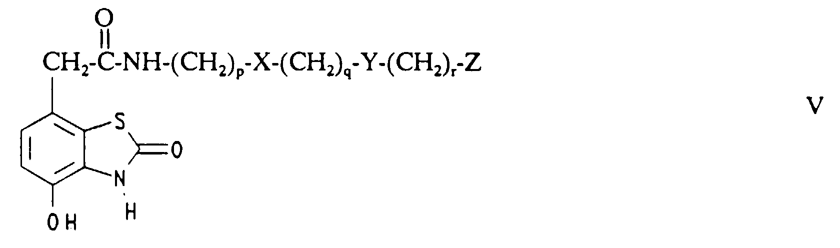 in which p, q, r, X, Y and Z are as defined above,
d) selective reduction of a compound of formula Va, 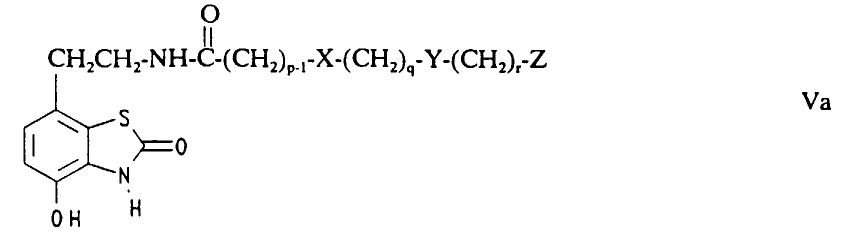 in which p, q, r, X, Y and Z are as defined above,
e) removal of a protecting group from a corresponding protected compound of formula I in which one or more of the functional groups is protected,
   and where desired or necessary converting the resulting compound of formula I to a pharmaceutically acceptable salt thereof, or vice versa.

In process a) leaving groups which L may represent include halide, such as chloride, bromide and iodide, and alkyl or arylsulphonyloxy groups such as methanesulphonyloxy or p-toluenesulphonyloxy. The reaction is preferably carried out in the presence of a base, e.g. an inorganic base such as sodium or potassium carbonate, or an organic base such as triethylamine, N,N-diisopropylethylamine or pyridine. The reaction is conveniently performed in a solvent such as an ether, e.g. tetrahydrofuran or dioxan, a ketone, e.g. butanone or methyl isobutyl ketone, a substituted amide, e.g. dimethylformamide, or a chlorinated hydrocarbon, e.g. chloroform, at a temperature of between ambient temperature and the reflux temperature of the solvent.

The alkylating agent of formula III may be prepared from the corresponding alcohol (i.e. the compound in which L represents OH) by known methods. For example, the alcohol may be reacted with a halogenating agent to yield the compound of formula III in which L represents a halogen atom. Suitable halogenating agents include, for example, triphenylphosphine-tetrahalogenomethane adduct (conveniently formed *in situ*, e.g. by the reaction of triphenylphosphine and carbontetrabromide). The reaction may take place in the presence of a solvent such as acetonitrile, or a chlorinated hydrocarbon, e.g. dichloromethane, at a temperature in the range of 0-30°C.

In process b) suitable reducing agents include hydrogen in the presence of a catalyst such as platinum, platinum oxide, palladium, palladium oxide, Raney nickel or rhodium, on a support such as charcoal, using an alcohol, e.g. ethanol, or an ester, e.g. ethyl acetate, or an ether, e.g. tetrahydrofuran, or water, as reaction solvent, or a mixture of solvents, at normal or elevated temperature and pressure. Alternatively the reducing agent may be a hydride such as diborane or a metal hydride such as sodium borohydride, sodium cyanoborohydride or lithium aluminium hydride. Suitable solvents for the reaction with these reducing agents will depend on the particular hydride used, but will include alcohols, such as methanol or ethanol, or ethers, such as diethyl ether or t-butyl methyl ether, or tetrahydrofuran.

Alkylation using the compound of formula IV may give rise to an intermediate imine, reduction of which under the conditions described yields the compound of formula I.

The compounds of formulae II and IV and the alcohols corresponding to formula III are either known or may be prepared by known techniques.

In processes c) and d) the reaction may be carried out using conventional reduction techniques. The reducing agent may be electrophilic, e.g. diborane, or nucleophilic, e.g. a complex metal hydride such as lithium aluminium hydride or sodium bis(2-methoxyethoxy)aluminium hydride. The solvent is preferably inert to the reaction conditions. Aprotic solvents are preferred, e.g. tetrahydrofuran, diethyl ether, or 1,2-dimethoxyethane. The reaction may be carried out at a temperature of from about 0 to 100°C.

The compounds of formulae V and Va may be prepared by coupling of an amine and an acid or acid chloride by conventional means. For example, the coupling may be performed in the presence of dicyclohexylcarbodiimide using the method of Sheehan and Hess, J. *Am. Chem. Soc.,* 1955, **77**, 1067; or 1,1-dicarbonyldiimidazole as described by Staab, *Angew. Chem. Int. Ed. Engl.,* 1962, **1**, 351. The amines required for the coupling reaction are either known or may be prepared by conventional methods, for example, as described in *J. Med. Chem.,* 1987, **30**, 1166.

The intermediates of formula Va are novel, thus according to a further aspect of the invention there are provided compounds of formula Va, 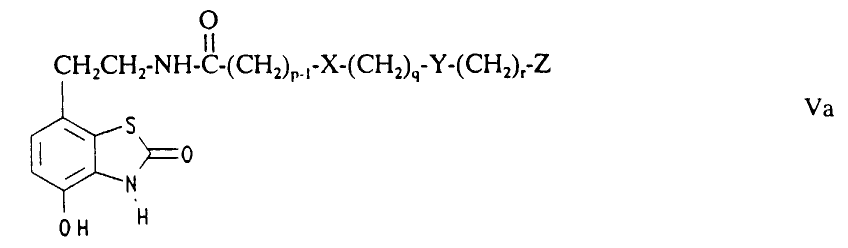 in which p, q, r, X, Y and Z are as defined above.

Further preparative details for the compounds of formula I are given in the Examples.

In the above processes it may be necessary for any functional groups, e.g. hydroxy or amino groups, present in the starting materials to be protected, thus process e) may involve the removal of one or more protecting groups. Suitable protecting groups and methods for their removal are, for example, those described in "Protective Groups in Organic Synthesis" by T.W. Greene and P.G.M. Wuts, John Wiley and Sons Inc., 1991. Hydroxy groups may, for example, be protected by arylmethyl groups such as phenylmethyl, diphenylmethyl or triphenylmethyl, or as tetrahydropyranyl derivatives.

Suitable amino protecting groups include arylmethyl groups such as benzyl, (R,S)-α-phenylethyl, diphenylmethyl or triphenylmethyl, and acyl groups such as acetyl, trichloroacetyl or trifluoroacetyl. Conventional methods of deprotection may be used. Arylmethyl groups may, for example, be removed by hydrogenolysis in the presence of a metal catalyst e.g. palladium on charcoal. Tetrahydropyranyl groups may be cleaved by hydrolysis under acidic conditions. Acyl groups may be removed by hydrolysis with a base such as sodium hydroxide or potassium carbonate, or a group such as trichloroacetyl may be removed by reduction with, for example, zinc and acetic acid.

Suitable pharmaceutically acceptable salts of the compounds of formula I include acid addition salts derived from inorganic and organic acids, such as hydrochlorides, hydrobromides, sulphates, phosphates, maleates, tartrates, citrates, benzoates, 4-methoxybenzoates, 2- or 4-hydroxybenzoates, 4-chlorobenzoates, benzenesulphonates, p-toluenesulphonates, naphthalenesulphonates, methanesulphonates, sulphamates, ascorbates, salicylates, acetates, diphenylacetates, triphenylacetates, adipates, fumarates, succinates, lactates, glutarates, gluconates, hydroxy-naphthalenecarboxylates, e.g. 1-hydroxy or 3-hydroxy-2-naphthalenecarboxylates, or oleates. The compounds may also form salts with suitable bases. Examples of such salts include alkali metal, e.g. sodium and potassium, and alkaline earth metal, e.g. calcium and magnesium, salts. The compound of formula I may be obtained in the form of a salt, conveniently a pharmaceutically acceptable salt. Where desired, such salts may be converted to the free bases using conventional methods. Pharmaceutically acceptable salts may be prepared by reacting the compound of formula I with an appropriate acid or base in the presence of a suitable solvent.

Suitable pharmaceutically acceptable esters of the compounds of formula I include alkyl C₁₋₆ esters, e.g. ethyl ester. The esters may be made by conventional techniques, e.g. esterification or transesterification.

Suitable amides include unsubstituted or mono- or di-substituted alkyl C₁₋₆ or phenyl amides, and may be made by conventional techniques, e.g. reaction of an ester of the corresponding acid with ammonia or an appropriate amine.

The compounds of formula I may exhibit tautomerism, they may also contain one or more asymmetric carbon atoms and may therefore exhibit optical and/or diastereoisomerism. Diastereoisomers may be separated using conventional techniques, e.g. chromatography or fractional crystallisation. The various optical isomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation.

By the term alkyl we mean straight, branched or cyclic saturated or unsaturated alkyl groups.

When Z represents substituted phenyl we prefer it to be substituted by only one group. The phenyl may be substituted *ortho, meta or para* to the -(CH₂)ₚ-X-(CH₂)_{q}-Y-(CH₂)ᵣ- group, however, we prefer compounds in which the phenyl is substituted *ortho* or *para* to the -(CH₂)ₚ-X-(CH₂)_{q}-Y-(CH₂)ᵣ- group.

Z may represent pyridinyl or thienyl. However, we prefer compounds of formula I in which Z represents phenyl.

Halogens with which Z may be substituted include bromine, chlorine and fluorine.

We prefer compounds of formula I in which Z represents phenyl.

We prefer compounds of formula I in which at least one of X and Y represents -O-.

We prefer compounds of formula I in which r represents 2.

We prefer compounds of formula I in which p+q represents 5

Specific groups which -(CH₂)ₚ-X-(CH₂)_{q}-Y-(CH₂)ᵣ- may represent include the following:

-(CH₂)₃-S-(CH₂)₂-O-(CH₂)₂-

-(CH₂)₃-SO₂-(CH₂)₂-O-(CH₂)₂-

-(CH₂)₃-O-(CH₂)₂-O-(CH₂)₂-

-(CH₂)₂-O-(CH₂)₃-O-(CH₂)₂-

-(CH₂)₂-O-(CH₂)₂-O-(CH₂)₂-

-(CH₂)₂-SO₂-(CH₂)₃-O-(CH₂)₂-

-(CH₂)₂-S-(CH₂)₃-O-(CH₂)₂-

The compounds of formula I are useful in that they exhibit pharmacological activity in animals. In particular the compounds are β₂-adrenoreceptor agonists. The activity may be demonstrated in the isolated trachea of the guinea pig, as described by I.G. Dougall, D. Harper, D.M. Jackson, and P. Leff, *Br. J. Pharmacol.,* 1991, **104**, 1057. The compounds are also dopamine DA₂-agonists. The binding affinities of the test compounds for the DA₂ binding sites in bovine pituitary membranes may be determined from the displacement of [³H]-N-n-propylnorapomorphine and of [³H]-spiperone in the absence or presence of nonhydrolysable GTP analogue respectively, D.R. Sibley, A. DeLean and I. Creese, Anterior Pituitary Dopamine Receptors, Demonstration of Interconvertible High and Low Affinity States of the D-2 Dopamine Receptor, *J. Biol. Chem.,* 1982, **257**(11), 6351-6361. The DA₂ activity may also be demonstrated in a functional screen, the rabbit isolated ear artery, as described by Brown and O'Connor, *Br. J. Pharmacol.,* 1981, **73**, 189P. The compounds also show advantageous DA₂:β₂ activity ratios.

The compounds of formula I are indicated for use in the treatment of the range of conditions known as reversible obstructive airways disease. The term "reversible obstructive airways disease" will be well understood by those skilled in the art to include conditions such as asthma, including bronchial asthma, allergic asthma, intrinsic asthma, extrinsic asthma and dust asthma, particularly chronic or inverterate asthma (for example late asthma and airway hyper-responsiveness); bronchitis and the like (see, for example, UK Patent No. 2022078 and *Br. J. Pharmacol.,* 1987, **24**, 4983). Of particular interest is asthma.

The term "treatment" as used herein includes prophylaxis as well as relieving the symptoms of disease.

According to another aspect of the invention there is therefore provided a method of treatment or prophylaxis of reversible obstructive airways disease, which method comprises administering a therapeutically effective quantity of a compound of formula I, or a pharmaceutically acceptable salt thereof, to a patient suffering from or susceptible to such a condition.

The compounds of formula I are also indicated for use in the treatment of various other conditions, e.g. inflammatory and allergic skin disorders, congestive heart failure and glaucoma.

For the above mentioned uses the doses administered will, of course, vary with compound employed, the mode of administration and the treatment desired. However, in general, satisfactory results are obtained when the compound of formula I is administered at a daily dosage of from about 1 µg to about 20 mg per kg of animal body weight, preferably given in divided doses 1 to 4 times a day or in sustained release form. For man the total daily dose is in the range of from 70 µg to 1,400 mg and unit dosage forms suitable for administration comprise from 20 µg to 1,400 mg of the compound admixed with a solid or liquid pharmaceutical diluent or carrier.

The compounds of formula I may be used on their own or in the form of appropriate pharmaceutical compositions for topical, enteral or parenteral administration.

Compositions in a form suitable for topical administration to the lung include aerosols, e.g. pressurised or non-pressurised powder compositions;
compositions in a form suitable for oesophageal administration include tablets, capsules and dragees;
compositions in a form suitable for administration to the skin include creams, e.g. oil-in-water emulsions or water-in-oil emulsions;
compositions in a form suitable for administration intravenously include injections and infusions; and
compositions in a form suitable for administration to the eye include drops and ointments.

According to the invention there is also provided a pharmaceutical composition comprising, preferably less than 80% and more preferably less than 50% by weight of, a compound of formula I. or a pharmaceutically acceptable derivative thereof, in admixture with a pharmaceutically acceptable diluent or carrier.

Examples of such diluents and carriers are:
for tablets and dragees - lactose, starch, talc, stearic acid;
for capsules - tartaric acid or lactose; and
for injectable solutions - water, alcohols, glycerin, vegetable oils.

When the compound of formula I is to be administered to the lung it may be inhaled as a powder which may be pressurised or non-pressurised. Pressurised powder compositions of the compounds of formula I may contain a liquified gas propellant or a compressed gas. In non-pressurised powder compositions the active ingredient in finely divided form may be used in admixture with a larger-sized pharmaceutically acceptable carrier comprising particles of up to, for example, 100 µm in diameter. Suitable inert carriers include, e.g. crystalline lactose.

The compounds of formula I have the advantage that they are less toxic, more efficacious, are longer acting, have a broader range of activity, are more potent, produce fewer side effects, are more easily absorbed or have other useful pharmacological properties, than compounds of a similar structure.

The invention is illustrated, but in no way limited, by the following Examples, in which temperatures are in degrees Celsius. The reactions were performed under an inert atmosphere of either nitrogen or argon. Preparative HPLC separations were generally performed using a DYNAMAX™ 60A C-18 reverse phase column.

### Example 1

### 4-Hydroxy-7-[2-[3-[2-[2-phenylethoxy]ethylsulphonyl]propylamino]ethyl]-1,3-benzothiazol-2(3H)-one hydrochloride

a) 3-[2-[2-Phenylethoxy]ethylthio]propanoic acid
   A solution of 2-[2-phenylethoxy]ethane thiol (2.13 g) in dry DMF (10 ml) was added dropwise to a cooled (0°) stirred suspension of sodium hydride (0.60 g, 80% in oil) in DMF (50 ml). The mixture was stirred at 0° for 90 minutes. A solution of 3-bromopropanoic acid (3.15 g) in dry DMF (10 ml) was then added dropwise and the reaction was stirred at room temperature for 16 hours. Water (250 ml) was added and the whole was acidified to pH 2/3 with concentrated hydrochloric acid. The aqueous solution was extracted several times with ether and the combined ethereal layers were washed with water and brine, dried (MgSO₄) and evaporated under reduced pressure. This yielded the crude acid which was purified by flash chromatography over silica using 6:1 dichloromethane:ether (1 drop acetic acid/100 ml of eluant) to give the sub-titled compound (2.15 g).
   ¹H NMR (CDCl₃) δ: 2.6-2.8 (m, 4H), 2.81 (t, 2H), 2.89 (t, 2H), 3.6-3.76 (m, 4H), 7.2-7.4 (m, 5H).
b) 3-[2-[2-Phenylethoxy]ethylsulphonyl]propanoic acid
   A solution of potassium peroxymonosulphate (15.6 g, OXONE™) in water (50 ml) was added dropwise to a cooled (0°) solution of the material from step a) (2.15 g) in methanol (50 ml). After the addition was complete the ice bath was removed and the reaction stirred at room temperature for 4 hours. The whole was poured into water and extracted three times with chloroform. The combined organic extracts were washed with water, dried (MgSO₄) and evaporated under reduced pressure to give the sub-titled compound as a white solid (1.91 g, 79%).
   Mass Spectrum: EI TMS derivative 343 [(M-15)⁺];
   ¹H NMR (CDCl₃) δ: 2.76 (t, 2H), 2.91 (t, 2H), 3.19 (m, 4H), 3.72 (t, 2H), 3.86 (t, 2H), 7.15-7.3 (m, 5H).
c) N-[2-[4-Hydroxy-2-oxo-3H-1,3-benzothiazol-7-yl]ethyl]-3-[2-[2-phenylethoxy]ethylsulphonyl]propanamide
   Triethylamine (0.70 ml), 1-hydroxybenzotriazole hydrate (0.98 g) and finally dicyclohexylcarbodiimide (1.49 g) were added to a stirred solution of 7-[2-aminoethyl]-4-hydroxy-1,3-benzothiazol-2(3H)-one hydrobromide (1.62 g) and the material from step b) (1.75 g) in DMF (25 ml). The whole was stirred for 16 hours at room temperature. Glacial acetic acid (0.1 ml) was added and stirring continued for 15 minutes. The DMF was removed under reduced pressure and the residue slurried with ethyl acetate (50 ml). The suspended dicyclohexylurea was removed by filtration. The filtrate was washed with saturated aqueous sodium bicarbonate and dried (MgSO₄). The solvent was removed under reduced pressure to yield a residue which was purified by column chromatography over silica using 95:5 dichloromethane:ethanol to give the sub-titled compound (1.89 g, 71%).
   mp 142-144°;
   Mass Spectrum: FAB +ve 479 [(M+H)⁺];
   ¹H NMR (D₆-DMSO) δ: 2.50 (m, 2H), 2.61 (t, 2H), 2.81 (t, 2H), 3.2-3.4 (brm, 6H + D₂O), 3.64 (t, 2H), 3.75 (t, 2H), 6.70 (d, 1H), 6.80 (d, 1H), 7.15-7.30 (m, 5H), 8.14 (t, 1H), 10.0 (brs, 1H), 11.5 (s, 1H);
   Analysis: Found; C,55.03; H,5.55; N.5.90; S,13.07%,
   C₂₂H₂₆N₂O₆S₂ requires: C,55.21; H,5.48; N,5.85; S,13.39%.
d) 4-Hydroxy-7-[2-[3-[2-[2-phenylethoxy]ethylsulphonyl]propylamino]ethyl]-1,3-benzothiazol-2(3H)-one hydrochloride
   Borane-tetrahydrofuran solution (1.0 M in THF, 15 ml) was added dropwise to a stirred solution of the product from step c) (2.06 g) in dry tetrahydrofuran (100 ml). The reaction was refluxed under an inert atmosphere until thin layer chromatography indicated that no more starting material remained. The reaction was cooled and methanol (3.5 ml) was added (CAUTION !). The reaction was refluxed for a further 30 minutes. The solvents were removed under reduced pressure and the residue dissolved in methanol (100 ml) to which was added concentrated hydrochloric acid (sg. 1.18, 0.75 ml). This was refluxed for 30 minutes. Cooling and removal of the methanol under reduced pressure yielded an oily residue which when triturated with ether gave the crude title compound as a pale yellow solid. Portions of the title compound were purified by preparative reverse phase HPLC using methanol and 0.1% trifluoroacetic acid as eluant. Finally preparation of the hydrochloride salt, by dissolving in a small amount of ethanol and treatment with dry ethereal hydrochloric acid followed by removal of the solvents, gave the title compound as a white powder.
   mp 201-203°;
   Mass Spectrum: FAB +ve 465 [(M+H)⁺];
   ¹H NMR (D₆-DMSO) δ: 2.01 (m, 2H), 2.80 (m, 4H), 2.98 (brs, 2H), 3.10 (t, 4H), 3.36 (t, 2H), 3.66 (t, 2H), 3.77 (t, 2H), 6.77 (d, 1H), 6.88 (d, 1H), 7.2-7.35 (m, 5H), 8.98 (brs, 2H), 10.13 (brs, 1H), 11.77 (s, 1H);
   Analysis: Found; C,52.31; H,5.85; N,5.54; S,12.54; Cl,7.48%,
   C₂₂H₂₈N₂O₅S₂.HCl requires: C,52.73; H,5.83; N,5.90; S,12.79; Cl,7.08%.

### Example 2

### 4-Hydroxy-7-[2-[2-[3-[2-phenylethoxy]propoxy]ethylamino]ethyl]-1,3-benzothiazol-2(3H)one hydrochloride

a) 2-[3-[2-Phenylethoxy]propoxy]acetic acid
   The sub-titled compound was prepared following the general method outlined in Example 1a) using 3-[2-phenylethoxylpropanol (prepared from 2-phenylmethyl-1,3-dioxane following the method described in *Can. J. Chem.,* 1974, **52**, 888).
   ¹H NMR (CDCl₃) δ: 1.89 (m, 2H), 2.90 (q, 2H), 3.49-3.60 (m, 6H), 4.05 (s, 2H), 7.21-7.30 (m, 5H).
b) N-[2-[4-Hydroxy-2-oxo-3H-1,3-benzothiazol-7-yl)ethyl)-2-[3-[2- phenylethoxy]propoxy]acetamide
   The sub-titled compound was prepared according to the general method outlined in Example 1c).
   mp 150-151°;
   Mass Spectrum: FAB +ve 431 [(M+H)⁺];
   ¹H NMR (D₆-DMSO) δ: 1.73 (m, 2H), 2.63 (t, 2H), 2.79 (t, 2H), 3.2-3.4 (brm, 6H + D₂O), 3.54 (t, 2H), 3.76 (brs, 2H), 6.69 (d, 1H), 6.79 (d, 1H), 7.16-7.29 (m, 5H), 8.12 (t, 1H), 9.92 (s, 1H), 11.61 (s, 1H);
   Analysis: Found; C,60.90; H,6.02; N,6.40; S,6.91%,
   C₂₂H₂₆N₂O₅S requires: C,61.37; H,6.09; N,6.51; S,7.45%.
c) 4-Hydroxy-7-[2-[2-[3-[2-phenylethoxy]propoxy]ethylamino]ethyl]-1,3-benzothiazol-2(3H)one hydrochloride
   The title compound was prepared according to the general method outlined in Example 1d).
   mp 159-160°;
   Mass Spectrum: FAB +ve 417 [(M+H)⁺];
   ¹H NMR (D₆-DMSO) δ: 1.75 (t, 2H), 2.79 (t, 2H), 2.87 (t, 2H), 3.12 (m, 4H), 3.45 (m, 4H+D₂O), 3.58 (m, 4H), 6.77 (d, 1H), 6.85 (d, 1H), 7.18-7.27 (m, 5H), 8.99 (brs, 2H), 10.16 (s, 1H), 11.8 (brs, 1H);
   Analysis: Found; C,58.33; H,6.54; N,6.37; S,6.79; Cl,7.96%,
   C₂₂H₂₈N₂O₄S.HCl requires: C,58.33; H,6.23; N,6.18; S,7.08; Cl,7.83%.

### Example 3

### 4-Hydroxy-7-[2-[3-[2-[2-phenylethoxy]ethoxy]propylamino]ethyl]-1,3-benzothiazol-2(3H)one hydrochloride

a) 3-[2-[2-Phenylethoxy]ethoxy]propanenitrile
   A mixture of 3-(2-phenylethoxy)ethanol (8.0 g, prepared from 2-phenylmethyl 1,3-dioxolane according to the general method in *Can. J. Chem.,* 1974, 52, 888), 3-bromopropanenitrile (5.6 ml), sodium hydroxide (50 g) and tetrabutylammonium chloride (0.5 g) in dichloromethane (100 ml) and water (100 ml) was stirred at room temperature for 72 hours. The mixture was diluted with water and the organic layer separated. The aqueous layer was extracted with a further portion of dichloromethane. The combined organic extracts were washed with dilute aqueous hydrochloric acid and water, dried (MgSO₄) and evaporated under reduced pressure to yield the crude product. This material was purified by flash chromatography over silica using 1:1 ether:petroleum ether (bp 60-80°) as eluant to give the sub-titled compound as an oil (9.84 g, 90%).
   Mass Spectrum: EI 219 (M)⁺;
   ¹H NMR (CDCl₃) δ: 2.55 (t, 2H), 2.90 (t, 2H), 3.61-3.74 (m, 8H), 7.18-7.36 (m, 5H).
b) 3-[2-[2-Phenylethoxy]ethoxy]propanal
   Diisobutylaluminum hydride (3.3 ml, 1.5 M in toluene) was added dropwise to a cooled (0°) stirred solution of 3-[2-[2-phenylethoxy]ethoxy]propanenitrile (1.0 g) from step a) in tetrahydrofuran. After 30 minutes the mixture was warmed to room temperature and stirred for a further 2 hours. Water and 10% aqueous hydrochloric acid were added cautiously and the reaction stirred for a further 5 minutes. The reaction was extracted several times with ether and the combined ethereal extracts were washed with saturated aqueous sodium bicarbonate solution and brine, dried (MgSO₄) and evaporated under reduced pressure to yield the sub-titled compound as a yellow oil. The material was used in the next step without purification.
c) 4-Hydroxy-7-[2-[3-[2-[2-phenylethoxy]ethoxy]propylamino]ethyl]-1,3-benzothiazol-2(3H)one hydrochloride
   Sodium cyanoborohydride (0.333 g) was added to a stirred solution of 3-[2-[2-phenylethoxy]ethoxy]propanal from step b) (2.2 g), 6% aqueous acetic acid (2 ml) and 7-[2-aminoethyl]-4-hydroxy-1,3-benzothiazol-2(3H)-one hydrobromide (2.05 g) in methanol (180 ml). The reaction was stirred for 2 hours at room temperature by which time HPLC analysis revealed that all the starting material had been consumed. The reaction was made basic with concentrated aqueous ammonium hydroxide solution and the methanol was removed under reduced pressure to yield the crude product. Purification by chromatography over silica using methanol in chloroform as the eluant, reverse phase preparative HPLC in methanol 0.1% aqueous trifluoroacetic acid as eluant and finally formation of the hydrochloride salt gave the title compound as a white solid.
   mp 186-190°;
   Mass Spectrum: FAB +ve 417 [(M+H)⁺];
   ¹H NMR (D₆-DMSO) δ: 1.80-1.88 (m, 2H), 2.78-2.86 (m, 4H), 2.97 (t, 2H), 3.09 (t, 2H), 3.46 (t, 2H), 3.47-3.58 (m, 4H), 3.60 (t, 2H), 6.76 (d, 1H), 6.88 (d, 1H), 7.16-7.29 (m, 5H), 8.70 (brs, 2H), 10.13 (s, 1H), 11.76 (brs, 1H);
   Analysis: Found; C,55.24; H,5.98; N,5.92; S,6.36; Cl,7.35%,
   C₂₂H₂₈N₂O₄S.HCl.1.42 H₂O requires: C,55.20; H,6.41; N,5.88; S,6.70; Cl,7.41%.

### Example 4

### 4-Hydroxy-7-[2-[2-[2-[2-phenylethoxy]ethoxy]ethylamino]ethyl]-1,3-benzothiazol-2(3H)-one hydrochloride

a) 2-[2-[2-Phenylethoxy]ethoxy]acetic acid
   Sodium hydride (60% dispersion in oil, 0.86 g) was washed several times with petroleum ether and suspended in tetrahydrofuran (5 ml). A solution of 2-[2-phenylethoxy]ethanol (1.5 g, prepared from 2-phenylmethyl-1,3-dioxolane according to the general method in *Can. J. Chem.,* 1974, **52**, 888) in tetrahydrofuran (10 ml) was added dropwise to the suspension and the mixture heated to 55° for 15 minutes and then stirred at room temperature for 2 hours. Chloroacetic acid (0.85 g) in tetrahydrofuran (5 ml) was added and stirring was continued for 17 hours at room temperature. The tetrahydrofuran was removed under reduced pressure and the residue partitioned between saturated aqueous sodium bicarbonate and diethyl ether (the ethereal layer was discarded). The separated aqueous layer was acidified using aqueous dilute hydrochloric acid and extracted with diethyl ether. The organic extracts were washed with saturated brine, dried (MgSO₄) and evaporated under reduced pressure to give a pale brown oil (1.48 g). Chromatography over silica using 1:1 ether petroleum ether (bp 60-80°) as eluant yielded the sub-titled compound (1.07 g).
   ¹H NMR (CDCl₃) δ: 2.94 (t, 2H), 3.49-3.82 (m, 6H), 4.16 (s, 2H), 7.18-7.34 (m, 5H).
b) N-[2-[4-Hydroxy-2-oxo-3H-1,3-benzothiazol-7-yl]ethyl]-2-[2-[2-phenylethoxy]ethyl] acetamide
   The sub-titled compound was prepared according to the general method outlined in Example 1c).
   Mass Spectrum: FAB +ve 417 [(M+H)⁺];
   ¹H NMR (D₆-DMSO) δ: 2.61 (t, 2H), 2.79 (t, 2H), 3.31 (m, 6H), 3.60 (t, 2H), 3.82 (s, 2H), 6.69 (d, 1H), 6.79 (d, 1H), 7.15-7.29 (m, 5H), 7.72 (t, 1H), 9.91 (brs, 1H), 11.61 (brs, 1H).

### c) 4-Hydroxy-7-[2-[2-[2-[2-phenylethoxy]ethoxy]ethylamino]ethyl]-1,3-benzothiazol-2(3H)-one hydrochloride

The title compound was prepared using the general method outlined in Example 1d).
mp 123°;
Mass Spectrum: FAB +ve 403 [(M+H)⁺];
¹H NMR (D₆-DMSO) δ: 2.78 (t, 2H), 2.85 (t, 2H), 3.09 (m, 4H), 3.56 (m, 6H), 3.65 (t, 2H), 6.77 (d, 1H), 6.83 (d, 1H), 7.08-7.29 (m, 5H), 9.00 (s, 2H), 10.15 (s, 1H), 11.69 (s, 1H);
Analysis: Found; C,56.62; H,6.15; N,6.43; Cl,9.40%,
C₂₁H₂₆N₂O₄S.HCl excess 0.18 moles HCI requires: C,56.62; H,6.14; N,6.29; Cl,9.37%.

### Example 5

### 4-Hydroxy-7-[2-[2-[3-[2-phenylethoxy]propylthio]ethylamino]ethyl]-1,3-benzothiazol-2(3H)-one hydrochloride

a) 3-Mercaptopropanol
   A solution of thiourea (36 g) in water (100 ml) was mixed with 3-bromopropanol (33 ml) and refluxed for 4 hours. The mixture was allowed to cool sligh:!y and 10% aqueous sodium hydroxide solution (190 ml) added. The mixture was again heated under reflux for a further 3 hours, allowed to cool and left to stand for 17 hours at room temperature. The reaction mixture was acidified to pH 4 using concentrated sulphuric acid and extracted with diethyl ether. The combined organic extracts were dried (MgSO₄) and concentrated under reduced pressure to give the crude product as a yellow liquid. Distillation gave the sub-titled compound (14.67 g).
   Mass Spectrum: EI 92 (M)⁺;
   ¹H NMR (CDCl₃) δ: 1.40 (m, 1H), 1.91 (m, 3H), 2.63 (q, 2H), 3.75 (t, 2H).
b) 2-Phenylmethyl-1,3-oxathiane
   To a solution of the thiol (14.67 g) from step a) in toluene (200 ml) was added p-toluenesulphonic acid (1 g) and phenylacetaldehyde (18.3 ml). The reaction was refluxed using a Dean and Stark apparatus. After the appropriate amount of water had been collected the reaction mixture was cooled, washed with saturated sodium bicarbonate, saturated brine and dried (K₂CO₃). The crude product was distilled (bp 100-110°/0.3 mbars) to give a yellow liquid (19.65 g).
   Mass Spectrum: EI 194 (M)⁺;
   ¹H NMR (CDCl₃) δ: 1.66 (d, 1H), 1.95 (m, 1H), 2.7 (m, 1H), 2.94 (m, 2H), 3.10 (m, 1H), 3.53 (t, 1H), 4.14 (d, 1H), 4.90 (t, 1H), 6.69-7.32 (m, 5H).
c) 3-[2-Phenylethoxy]propanethiol
   Calcium turnings (3.5 g) were added portionwise to liquid ammonia (500 ml) and the whole was stirred vigorously for 10 minutes. The thioacetal from step b) (10 g) in ether (7 ml) was added dropwise to the dark blue solution over a 7 minute period. The mixture was stirred for 2 hours and then quenched with ammonium chloride until effervescing had ceased. Excess ammonia was allowed to evaporate by purging under nitrogen overnight. The remaining solid was acidified using dilute 10% aqueous hydrochloric acid to pH 1-2 and the product extracted with ethyl acetate. The organic layers were combined, washed with water and brine, dried (MgSO₄) and concentrated under reduced pressure to give the sub-titled compound (8.29 g).
   Mass Spectrum: EI 196 (M)⁺;
   ¹H NMR (CDCl₃) δ: 1.29 (d, 1H), 1.86 (m, 2H), 2.56 (q, 2H), 2.87 (t, 2H), 3.49 (t, 2H), 3.64 (t, 2H), 6.97-7.31 (m, 5H).
d) 2-[3-[2-Phenylethoxy]propylthio]acetic acid
   Sodium hydride (60%, 3.38 g) was washed with petroleum ether and suspended in dimethylformamide (5 ml) at 0°. A solution of the thiol from step c) in dimethylformamide (8.29 g in 10 ml) was added dropwise. Stirring was continued for 2 hours at 0-8° at which point a solution of bromoacetic acid (5.88 g) in dimethylformamide (15 ml) was added dropwise. A further quantity of dimethylformamide (20 ml) was added to aid stirring. After 17 hours at room temperature the dimethylformamide was removed under reduced pressure. The residue was partitioned between saturated aqueous sodium bicarbonate and diethyl ether (the ethereal layer was discarded). The aqueous layer was separated, acidified with hydrochloric acid to pH 1-2 and extracted with diethyl ether. The ethereal extracts were combined, washed with water and brine, dried (MgSO₄) and concentrated under reduced pressure. Chromatography of the crude material over silica using 1:1 petroleum ether (bp 60-80°):ether as eluant gave the sub-titled compound (7.10 g).
   ¹H NMR (CDCl₃) δ: 1.86 (m, 2H), 2.70 (t, 2H), 2.87 (t, 2H), 3.21 (s, 2H), 3.51 (t, 2H), 3.63 (t, 2H), 7.17-7.30 (m, 5H), 9.74 (s, 1H).
e) N-[2-[4-Hydroxy-2-oxo-3H-1,3-benzothiazol-7-yl]ethyl]-2-[3-[2-phenylethoxy]propylthio]acetamide
   The sub-titled compound was prepared using the general method outlined in Example 1c) using 7-[2-aminoethyl]-4-hydroxy-1,3-benzothiazol-2(3H)-one hydrobromide to yield, after purification by chromatography over silica using 9:1 dichloromethane:ethanol as eluant, the sub-titled compound (1.08 g).
   Mass Spectrum: FAB +ve 447 [(M+H)⁺];
   ¹H NMR (D₆-DMSO) δ: 1.70 (m, 2H), 2.65 (t, 2H), 2.67 (t, 2H), 2.78 (t, 2H), 3.05 (s, 2H), 3.28 (q, 2H), 3.41 (t, 2H), 3.53 (t, 2H), 6.71 (d, 1H), 6.83 (d, 1H), 7.15-7.43 (m, 5H), 8.05 (s, 1H), 9.9 (s, 1H), 11.62 (s, 1H).
f) 4-Hydroxy-7-[2-[2-[3-[2-phenylethoxy]propylthio]ethylamino]ethyl]-1,3-benzothiazol-2(3H)-one hydrochloride
   The title compound was prepared according to the general method outlined in Example 1d). The crude product was purified by reverse phase HPLC using 0.1% aqueous trifluoroacetic acid methanol as eluant.
   mp 209-211°;
   Mass Spectrum: FAB +ve 433 [(M+H)⁺];
   ¹H NMR (D₆-DMSO) δ: 1.82 (m, 2H), 2.62 (m, 4H), 2.87 (m, 4H), 2.93 (m, 2H), 3.16 (m, 2H), 3.53 (t, 2H), 3.65 (t, 2H), 6.83 (d, 1H), 6.94 (d, 1H), 7.26-7.37 (m, 5H), 9.02 (s, 2H), 10.21 (s, 1H), 11.83 (s, 1H);
   Analysis: Found; C,55.36; H,6.35; N,6.12; S,13.30%,
   C₂₂H₂₈N₂O₃S₂.HCl excess 0.46 moles H₂O requires: C,55.36; H,6.32; N,5.87; S,13.41%.

### Example 6

### 4-Hydroxy-7-[2-[2-[3-[2-phenylethoxy]propylsulphonyl]ethylamino]ethyl]-1,3-benzothiazol-2(3H)-one hydrochloride

a) 2-[3-[2-Phenylethoxy]propylsulphonyl]acetic acid
   The sub-titled compound was prepared from 2-[3-[2-phenylethoxy]propanethio]acetic acid [Example 5d)] using the general method described in Example 1b).
   Mass Spectrum: FAB +ve 287 [M + H]⁺;
   ¹H NMR CDCl₃ δ: 2.12 (m, 2H), 2.87 (t, 2H), 3.41 (t, 2H), 3.58 (t, 2H), 3.67 (t, 2H), 3.97 (s, 2H), 7.00-7.43 (m, 5H), 8.79 (s, 1H).
b) N-[2-[4-Hydroxy-2-oxo-3H-1,3-benzothiazole-7-yl]ethyl]-2-[3-[2-phenylethoxy]propylsulphonyl]acetamide
   The title compound was prepared according to the general method outlined in Example 1c). The crude material was purified by flash chromatography over silica using 9:1 dichloromethane:methanol as eluant.
   Mass Spectrum: FAB +ve 479 [(M+H)⁺];
   ¹H NMR (D₆-DMSO) δ: 1.92 (q, 2H), 2.62 (t, 2H), 2.81 (t, 2H), 3.27 (m, 4H), 3.49 (t, 2H), 3.58 (t, 2H), 4.04 (s, 2H), 6.70 (d, 1H), 6.83 (d, 1H), 7.17-7.29 (m, 5H), 8.47 (t, 1H), 9.96 (s, 1H), 11.66 (d ,1H).
c) 4-Hydroxy-7-[2-[2-[3-[2-phenylethoxy]propylsulphonyl]ethylamino]ethyl]-1,3-benzothiazol-2(3H)-one hydrochloride
   The title compound was prepared by the general method outlined in Example 1d). The crude material was purified by reverse phase HPLC using 0.1% aqueous trifluoroacetic acid methanol as eluant.
   mp 217-220°;
   Mass Spectrum: FAB +ve 465 [(M+H)⁺];
   ¹H (D₆-DMSO) δ: 1.91 (quin, 2H), 2.81 (t, 2H), 2.87 (t, 2H), 3.20 (m, 4H), 3.34 (t, 2H), 3.51 (t, 2H). 3.57 (q. 4H), 6.77 (d, 1H), 6.86 (d, 1H), 7.17-7.31 (m, 5H), 9.27 (s, 2H), 10.15 (s, 1H), 11.77 (s, 1H);
   Analysis: Found; C,52.57; H,6.05; N,5.73; S,12.61%,
   C₂₂H₂₈N₂O₅S₂.HCl requires: C,52.73; H,5.83; N,5.59; S,12.79%.

### Example 7

### 4-Hydroxy-7-[2-[3-[2-[2-phenylethoxy]ethylthio]propylamino]ethyl]-1,3-benzothiazol-2(3H)one hydrochloride

a) N-[2-[4-Hydroxy-2-oxo-3H-1,3-benzothiazol-7-yl]ethyl]-3-[2-[2-phenylethoxy]ethylthio]propanamide
   The sub-titled compound was prepared using the general method outlined in Example 1c). Using the compound from Example 1a).
   Mass Spectrum: FAB +ve 447 [(M+H)⁺];
   ¹H NMR (D₆-DMSO) δ: 2.26-2.33 (t, 2H), 2.54-2.72 (m, 6H), 2.75-2.83 (t, 2H), 3.19-3.28 (q, 2H), 3.50-3.63 (2 x t, 4H), 6.68 (d, 1H), 6.78 (d, 1H), 7.15-7.3 (m, 5H), 7.95 (t, 1H), 9.89 (s, 1H), 11.60 (brs, 1H).
b) 4-Hydroxy-7-[2-[3-[2-[2-phenylethoxy]ethylthio]propylamino]ethyl]-1,3-benzothiazol-2(3H)one hydrochloride
   The sub-titled compound was prepared using the general method outlined in Example 1d).
   mp 211-213°;
   Mass Spectrum: FAB +ve 433 [(M+H)⁺];
   ¹H NMR (D₆-DMSO) δ: 1.85 (m, 2H), 2.59 (t, 2H), 2.65 (t, 2H), 2.81 (t, 2H), 2.85 (t, 2H), 2.97 (t, 2H), 3.08 (m, 2H), 3.56 (t, 2H), 3.61 (t, 2H), 6.76 (d, 1H), 6.87 (d, 1H), 7.17-7.30 (m, 5H), 8.9 (brs, 2H), 10.14 (s, 1H), 11.76 (s, 1H);
   Analysis: Found; C,56.49; H,6.40; N,6.12; S,13.78; Cl,7.98%,
   C₂₂H₂₈N₂O₃S₂.HCl requires: C,56.33; H,6.23; N,5.97; S,13.67; Cl,7.56%.

### Example 8

### 4-Hydroxy-7-[2-[3-[2-[2-phenylethoxy]ethylsulphonyl]propylamino]ethyl]-1,3-benzothiazol-2(3H)-one 4-methylbenzenesulphonate

a) 4-Hydroxy-7-[2-[3-[2-[2-phenylethoxy]ethylsulphonyl]propylamino]ethyl]-1,3-benzothiazol-2(3H)-one
   An aqueous solution (500 ml) of the title compound from Example 1 (4.9 g) was mixed with an excess of aqueous sodium hydrogen carbonate. The free base was extracted with chloroform and the combined extracts were washed with water and dried (MgSO₄), filtered and the chloroform removed under reduced pressure to yield the sub-titled compound as an off-white solid (4.22 g, 91%).
   mp 69-70°.
b) 4-Hydroxy-7-[2-[3-[2-[2-phenylethoxy]ethylsulphonyl]propylamino]ethyl]-1,3-benzothiazol-2(3H)-one 4-methylbenzenesulphonate
   A portion of the free base was dissolved in methanol and one molar equivalent of 4-methylbenzenesulphonic acid was added. The solution was evaporated under reduced pressure and the solid collected was recrystallised (methanol/water) to yield the title compound as white needles.
   mp 170-171°.

### Example 9

### 4-Hydroxy-7-[2-[3-[2-[2-phenylethoxy]ethylsulphonyl]propylamino]ethyl]-1,3-benzothiazol-2(3H)-one hemisuccinate

The title compound was prepared using the general method outlined in Example 8a) and b) using succinic acid.
mp 182-183°, lustrous white plates (recrystallised from methanol/water).

### Example 10

### 4-Hydroxy-7-[2-[3-[2-[2-phenylethoxy]ethylsulphonyl]propylamino]ethyl]-1,3-benzothiazol-2(3H)-one hexanoate

The title compound was prepared using the general method outlined in Example 8a) and b) using hexanoic acid.
mp 131-132°, white needles (recrystallised from methanol/water).

### Example 11

### 4-Hydroxy-7-[2-[3-[2-[2-phenylethoxy]ethylsulphonyl]propylamino]ethyl]-1,3-benzothiazol-2(3H)-one tartrate

The title compound was prepared using the general method outlined in Example 8a) and b) using tartaric acid.
mp 158-162°, (recrystallised from methanol/water).

### Example 12

### 4-Hydroxy-7-[2-[3-[2-[2-phenylethoxy]ethylsulphonyl]propylamino]ethyl]-1,3-benzothiazol-2(3H)-one 1-hydroxy-2-naphthoate (Xinafoate)

The title compound was prepared using the general method outlined in Example 8a) and b) using 1-hydroxy-2-naphthoic acid.
mp 176-177°, white needles (recrystallised from methanol/water).

### Example 13

### 4-Hydroxy-7-[2-[3-[2-[2-[2-aminophenyl]ethoxy]ethylsulphonyl]propylamino]ethyl]-1,3-benzothiazol-2(3H)-one dihydrochloride

a) Methyl 3-[2-[2-[2-nitrophenyl]ethoxy]ethylsulphonyl] propanoate
   Concentrated nitric acid (3.25 ml) was added dropwise over a half hour period to a stirred cooled (ice/salt) solution of methyl 3-[2-[2-phenylethoxy]ethylsulphonyl]propanoate (15.12 g) (prepared from the acid, which was itself prepared by the procedure outlined in Example 1b)) in trifluoroacetic acid. The reaction was allowed to warm to room temperature, stirred overnight, diluted with water and extracted several times with ethyl acetate. The combined organic extracts were washed with water and brine, dried (MgSO₄) and concentrated under reduced pressure to yield the crude mixture of isomeric methyl 3-[2-[2-[nitrophenyl]-ethoxy]ethylsulphonyl propanoates. The sub-titled compound was separated from the other isomers by normal phase preparative HPLC using 1:1 hexane:ethyl acetate as eluant.
   ¹H NMR (CDCl₃) δ: 2.80-2.84 (t, 2H), 3.17-3.24 (m, 4H), 3.32-3.36 (m, 2H), 3.71-3.78 (m, 2H), 3.84-3.87 (t, 2H), 7.36-7.41 (m, 2H), 7.54 (t, 1H), 7.91 (d, 1H).
b) 3-[2-[2-[2-Nitrophenyl]ethoxy]ethylsulphonyl] propanoic acid
   Lithium metal (0.59 g) was allowed to dissolve in methanol (200 ml). Water (100 ml) was added and then the compound from step a) (6.05 g) in methanol (50 ml) was added dropwise to the cooled (ice/salt) solution. The reaction was allowed to warm to room temperature and stirring was continued overnight. The solvent was removed under reduced pressure and the residual material diluted with water. The basic aqueous solution was washed with ethyl acetate (which was discarded), acidified to pH 2 (concentrated hydrochloric acid) and extracted with ethyl acetate. These extracts were combined, washed with water and brine, dried (MgSO₄) and the solvents evaporated under reduced pressure to yield the crude product which was further purified by flash chromatography over silica using 9:1 dichloromethane:methanol as eluant to yield the sub-titled compound.
   Mass Spectrum: TS 349 [(M+NH₄)⁺].
c) N-[2-[4-Hydroxy-2-oxo-3H-1,3-benzothiazol-7-yl]ethyl]-3-[2-[2-[2-nitrophenyl]ethoxy]ethylsulphonyl]propanamide
   The sub-titled compound was prepared using the general method outlined in Example 1c) using 7-[2-aminoethyl]-4-hydroxy-1,3-benzothiazol-2(3H)-one hydrobromide and the product from step b) to yield, after purification by chromatography over silica using 6% ethanol in chloroform as eluant the sub-titled compound.
   Mass Spectrum: FAB +ve 524 [(M+H)⁺];
   ¹H NMR (D₆-DMSO) δ: 2.5 (m, 4H), 2.60 (t, 2H), 3.09 (t, 2H), 3.24 (m, 4H), 3.68 (t, 2H), 3.74 (t, 2H), 6.70 (d, 1H), 6.80 (d, 1H), 7.47 (t, 1H), 7.55 (d, 1H), 7.62 (t, 1H), 7.91 (d, 1H), 8.14 (t, 1H), 9.91 (s, 1H), 11.62 (s, 1H).
d) N-[2-[4-Hydroxy-2-oxo-3H-1,3-benzothiazol-7-yl]ethyl]-3-[2-[2-[2-aminophenyl]ethoxy]ethylsulphonyl] propanamide
   Hydrazine hydrate (10 ml) was added dropwise to a stirred suspension of freshly washed Raney Nickel, the compound from step c) (2.21 g) and ethanol (50 ml). After the reaction was complete the Raney Nickel was filtered off (CAUTION FIRE HAZARD !) and the ethanol evaporated under reduced pressure. The residue was partitioned between water and dichloromethane and the aqueous layer further extracted with portions of dichloromethane. The combined extracts were washed with water and brine, dried (MgSO₄) and evaporated under reduced pressure to yield the crude product. The material was used without further purification.
   Mass Spectrum: FAB +ve 494 [(M+H)⁺];
   ¹H NMR (D₆-DMSO) δ: 2.4-2.8 (t, 8H), 3.2-3.4 (m, 6H), 3.58 (t, 2H), 3.76 (t, 2H), 6.46 (t, 1H), 6.59 (d, 1H), 6.70 (d, 1H), 6.80 (d, 1H), 6.86-6.93 (m, 2H), 8.16 (t, 1H), 9.93 (brs, 1H), 11.63 (s, 1H).
e) 4-Hydroxy-7-[2-[3-[2-[2-[2-aminophenyl]ethoxy]ethylsulphonyl]propylamino]ethyl]-1,3-benzothiazol-2(3H)-one dihydrochloride
   The title compound was prepared using the general method outlined in Example 1d) using the product from step d). The crude reaction product was purified by preparative reverse phase HPLC using acetonitrile 0.1% aqueous trifluoroacetic acid as eluant.
   mp 65° (softens);
   Mass Spectrum: FAB +ve 480 [(M+H)⁺];
   ¹H NMR (D₆-DMSO) δ: 2.00-2.08 (m, 2H), 2.8-3.3 (m, 10H) 3.39-3.45 (m. 2H), 3.71 (t, 2H), 3.81 (t, 2H), 4.5 (brs, 3H), 6.77 (d, 1H), 6.89 (d, 1H), 7.28-7.36 (m, 4H), 9.04 (brs, 2H), 10.15 (s, 1H), 11.6 (s, 1H).

### Example 14

### 4-Hydroxy-7-[2-[3-[2-[2-[4-nitrophenyl]ethoxy]ethylsulphonyl]propylamino]ethyl]-1,3-benzothiazol-2(3H)-one hydrochloride

a) N-[2-[4-Hydroxy-2-oxo-3H-1,3-benzothiazol-7-yl]ethyl]-3-[2-[2-[4-nitrophenyl]ethoxy]ethylsulphonyl]propanamide
   Following the general method outlined in Example 1c) using 7-[2-aminoethyl]-4-hydroxy-1,3-benzothiazol-2(3H)-one hydrobromide and 3-[2-[2-[4-nitrophenyl]ethoxy]ethylsulphonyl] propanoic acid [Example 13a)] gave the sub-titled compound after purification by chromatography over silica using 8% ethanol in dichloromethane as eluant.
   Mass Spectrum: FAB +ve 524 [(M+H)⁺];
   ¹H NMR (D₆-DMSO) δ: 2.45 (t, 2H), 2.60 (t, 2H), 2.97 (t, 2H), 3.2-3.4 (m, 6H), 3.69-3.77 (m, 4H), 6.70 (d, 1H), 6.80 (d, 1H), 7.53 (d, 2H), 8.12 (d, 3H), 9.91 (brs, 1H), 11.6 (brs, 1H).
b) 4-Hydroxy-7-[2-[3-[2-[2-[4-nitrophenyl]ethoxy]ethylsulphonyl]propylamino]ethyl]-1,3-benzothiazol-2(3H)-one hydrochloride
   The title compound was prepared using the general method outlined in Example 1d). The crude reaction product was purified by preparative reverse phase HPLC using acetonitrile in 0.1% aqueous trifluoroacetic acid as eluant.
   mp 75-78°;
   Mass Spectrum: FAB +ve 510 [(M+H)⁺];
   ¹H NMR (D₆-DMSO) δ: 2.01 (quin, 2H), 2.83 (t, 2H), 2.98 (t, 4H), 3.12 (t, 4H), 3.39 (t, 2H), 3.70-3.79 (m, 4H), 6.76 (d, 1H), 6.88 (d, 1H), 7.55 (d, 2H), 8.16 (d, 2H), 8.83 (brs, 2H), 10.13 (s, 1H), 11.76 (s, 1H).

### Example 15

### 7-[2-[2-[3-[2-[4-Fluorophenyl]ethoxy]propylsulphonyl]ethylamino]ethyl]-4-hydroxy-1,3-benzothiazol-2(3H)-one hydrochloride

a) 2-[4-Fluorophenyl]ethyl allyl ether
   2-[4-Fluorophenyl]ethanol (7.0 g) was added slowly to a stirred suspension of sodium hydride (1.25 g, 60% as dispersion in oil prewashed with petroleum ether bp 60-80°) in DMF (50 ml). The mixture was stirred for 30 minutes at room temperature. Allyl bromide (6.0 g) was then added slowly and the whole allowed to stir overnight. The reaction was partitioned between water and ether. The aqueous layer was further extracted with ether and the combined ethereal extracts were washed with water and dried (MgSO₄). The ether was removed under reduced pressure to yield the crude sub-titled compound as a colourless oil (8.7 g, 96%).
   Mass Spectrum: EI 180 (M⁺);
   ¹H NMR (CDCl₃) δ: 2.9 (t, 2H), 3.6 (t, 2H), 4.0 (t, 2H), 5.2 (m, 2H), 5.9 (m, 1H), 6.95 (m, 2H), 7.2 (m, 2H).
   This reaction was successfully repeated on a x5 scale.
b) 2-[3-[2-[4-Fluorophenyl]ethoxy]propylthio]acetic acid
   The compound from step a) (15 g) and thioglycolic acid (6.8 ml) were stirred together at room temperature in a conical flask exposed to the atmosphere for 2 hours at which point more thioglycolic acid (3.4 ml) was added. After a further ½ hour stirring the reaction was complete. The crude material was chromatographed over silica using 99:1 dichloromethane:acetic acid as eluant to yield the sub-titled compound as a colourless oil (19.69 g, 87%).
   Mass Spectrum: FAB +ve 273 [(M+H)⁺];
   ¹H NMR (CDCl₃) δ: 1.87 (m, 2H), 2.71 (t, 2H), 2.85 (t, 2H), 3.31 (d, 2H), 3.51 (t, 2H), 3.65 (t, 2H), 6.98 (m, 2H), 7.18 (m. 2H).
c) 2-[3-[2-[4-Fluorophenyl]ethoxy]propylsulphonyl]acetic acid
   A solution of potassium hydrogen carbonate (150 g) in water (500 ml) was added over a 20 minute period to a stirred mixture of the acid from step b) (39.5 g) in water (50 ml). An aqueous solution of OXONE™ (278 g in 400 ml) was added in portions and the reaction left to stir overnight. Water (1 L) was then added and the whole extracted with ether (to remove non acidic material). The aqueous layer was then acidified with 20% aqueous sulphuric acid and extracted three times with ether. The ethereal layers were combined, washed with water and brine, dried (MgSO₄) and concentrated under reduced pressure to yield a pale yellow oil (41.7 g). A white solid precipitated out from the oil on standing. The solid was removed by filtration and washed with small amounts of 1:1 ether:pentane to yield the sub-titled compound.
   mp 47-48°;
   Mass Spectrum: FAB +ve 305 [(M+H)⁺];
   ¹H NMR (CDCl₃) δ: 2.12 (m, 2H), 2.84 (t, 2H), 3.32 (m, 2H), 3.58 (t, 2H), 3.65 (t, 2H), 4.00 (s, 2H), 6.98 (m, 2H), 7.16 (m, 2H), 8.80 (brs, 1H).
d) N-[2-[4-Hydroxy-2-oxo-3H-1,3-benzothiazol-7-yl]ethyl]-2-[3-[2-[4-fluorophenyl]ethoxy]propylsulphonyl]acetamide
   Carbonyl diimidazole (1.94 g) was added to a stirred solution of the acid from step c) (3.64 g) in DMF (15 ml). Stirring was continued for 40 minutes at room temperature. To this solution was added 7-[2-aminoethyl]-4-hydroxy-1,3-benzothiazol-2(3H)-one hydrobromide (3.48 g) followed by triethylamine (1.7 ml). The whole was left overnight. The reaction mixture was then added slowly to a rapidly stirred mixture of 10% aqueous hydrochloric acid and ether (100 ml each). A pale yellow solid gradually settled out and was filtered off, washed with pentane and dried *in vacuo.* This provided the sub-titled compound as a buff coloured material which was used in the next step without further purification.
   Mass Spectrum: FAB +ve 497 [(M+H)⁺];
   ¹H NMR (D₆-DMSO) δ: 1.91 (m, 2H), 2.61 (t, 2H), 2.80 (t, 2H), 3.30 (m, 4H), 3.48 (t, 2H), 3.56 (t, 2H), 4.03 (s, 2H), 6.70 (d, 1H), 6.82 (d, 1H), 7.10 (t, 2H), 7.28 (m, 2H), 8.46 (t, 1H), 9.95 (s, 1H), 11.66 (s, 1H).
e) 7-[2-[2-[3-[2-[4-Fluorophenyl]ethoxy]propylsulphonyl]ethylamino]ethyl]-4-hydroxy-1,3-benzothiazol-2(3H)-one hydrochloride
   The title compound was prepared using the general method outlined in Example 1d) using the compound from step d). The crude reaction product was purified by preparative reverse phase HPLC using 35% THF in 0.1% aqueous trifluoroacetic acid as eluant.
   mp 240-245°;
   Mass Spectrum: FAB +ve 483 [(M+H)⁺];
   ¹H NMR (D₆-DMSO) δ: 1.91 (m, 2H), 2.80 (t, 2H), 2.85 (t, 2H), 3.14 -3.21 (m, 4H), 3.24 (2H + D₂O), 3.49 (t, 2H), 3.54 (q, 4H), 6.76 (d, 1H), 6.87 (d, 1H), 7.10 (t, 2H), 7.27 (t, 2H), 9.14 (s, 2H), 10.15 (s, 1H), 11.78 (s, 1H);
   Analysis: Found; C,50.58; H,5.62; N,5.61; S,12.26%,
   C₂₂H₂₇N₂FO₅S₂.HCl requires: C,50.91; H,5.44; N,5.40; S,12.36%.

### Example 16

### 4-Hydroxy-7-[2-[2-[3-[2-[2-thienyl]ethoxy]propylthio]ethylamino]ethyl]-1,3-benzothiazol-2(3H)one hydrochloride

The title compound was prepared according to the procedure outlined in Example 15 employing 2-thienylethanol, except that for the oxidation of Example 15c) the general procedure described in Example 1b) was employed.
mp 220-221°;
Mass Spectrum: FAB +ve 471 [(M+H)⁺];
¹H NMR (D₆-DMSO) δ: 1.90-1.98 (m, 2H), 2.53 (m, 2H), 2.85 (t, 2H), 3.03 (t, 2H), 3.16 (m, 2H), 3.25 (m, 2H), 3.37 (m, 2H). 3.53 (m, 2H), 3.60 (t, 2H), 6.76 (d, 1H), 6.88 (m, 2H), 6.94 (m, 1H), 7.33 (dd, 1H), 9.09 (brs, 2H), 10.14 (s, 1H), 11.78 (brs, 1H); Analysis: Found; C,46.67; H,5.51; N,5.68; S,18.42%,
C₂₀H₂₆N₂O₅S₃.HCl requires: C,47.37; H,5.37; N,5.52; S,18.97%.

### Example 17

### 4-Hydroxy-7-[2-[3-[2-[2-[2-pyridyl]ethoxy]ethylthio]propylamino]ethyl]-1,3-benzothiazol-2(3H)one dihydrochloride

a) 2-[2-[2-Bromoethylthio]ethyl]-1,3-dioxolane
   To a stirred cooled ( < 0°) solution of 2-[2-[1,3-dioxolan-2-yl]ethylthio]ethanol (13,6 g, prepared by the condensation between mercaptoethanol and 2-[2-bromoethyl]-1,3-dioxolane employing sodium hydride) in dry acetonitrile (150 ml) was added triphenylphosphine (20 g) and carbon tetrabromide (38 g). The solution was stirred for 4 hours. The solvent was removed under reduced pressure and the residue pre-absorbed onto silica. The material was flash chromatographed over silica using 10% ethylacetate/petroleum ether as eluant to afford the sub-titled compound as a clear oil (4.45 g). The material was used in the next step without further purification.
b) 2-[2-[2-[2-[2-Pyridyl]ethoxy]ethylthio]ethyl]-1,3-dioxolane
   The material from step a) (6.12 g), tetra-n-butylammonium hydrogen sulphate (1 g) and 2-pyridylethanol (2.85 ml) were stirred together with dichloromethane and 20 % aqueous sodium hydroxide each (20 ml) until gas chromatographic analysis indicated that the reaction had stopped. The organic layer was separated, washed with water and dried (MgSO₄). Removal of the solvents under reduced pressure yielded an oil. This material was further purified by flash chromatography over silica using 4:1 ethyl acetate:petroleum ether (bp 60-80°) as eluant to yield the sub-titled compound as a yellow oil (0.770 g).
   ¹H NMR (CDCl₃) δ: 1.92 (m, 2H), 2.65 (m, 4H), 3.07 (t, 2H), 3.62 (t, 2H), 3.85 (m, 4H), 3.95 (m, 2H), 4.94 (t, 1H), 7.13 (m, 1H), 7.22 (d, 1H), 7.60 (td, 1H), 8.53 (d, 1H).
c) 3-[2-[2-[2-Pyridyl]ethoxy]ethylthio]propanal
   The material from step b) (0.850 g) was dissolved in 80% formic acid (10 ml) and left to stand at room temperature for 22 hours. The mixture was partitioned between ether and water. The layers were separated and the aqueous layer extracted with ether. The ethereal extracts were combined, washed with brine, dried (MgSO₄) and evaporated under reduced pressure to yield the sub-titled compound as an oil (0.70 g).
   ¹H NMR (CDCl₃) δ: 2.69 (m, 4H), 2.87 (t, 2H), 3.06 (t, 2H), 3.64 (t, 2H), 3.85 (t, 2H), 7.13 (td, 1H), 7.21 (d, 1H), 7.60 (td, 1H), 8.53 (d, 1H), 9.74 (s, 1H).
d) 4-Hydroxy-7-[2-[3-[2-[2-[2-pyridyl]ethoxy]ethylthio]propylamino]ethyl]-1,3-benzothiazol-2(3H)one dihydrochloride
   The material from step c) (0.700 g) was dissolved in methanol (20 ml). To this solution was added 7-[2-aminoethyl]-4-hydroxy-1,3-benzothiazol-2(3H)-one hydrobromide (0.655 g), sodium cyanoborohydride (0.100 g) and aqueous 6% acetic acid (to adjust the pH to 6). The solution was stirred at room temperature overnight and then made basic with concentrated ammonium hydroxide solution. The solvent was removed under reduced pressure and the residual material purified by column chromatography over silica using methanol in dichloromethane as eluant. The resultant combined fractions were further purified by reverse phase HPLC using acetonitrile in 0.1% aqueous trifluoroacetic acid as eluant which after preparation of the hydrochloride salt yielded a pure sample of the title compound.
   mp 50-60° (softens);
   Mass Spectrum: FAB +ve 434 [(M+H)⁺];
   ¹H NMR (D₆-DMSO) δ: 1.86 (m, 2H), 2.55 (t, 2H), 2.62 (t, 2H), 2.88 (m, 2H), 2.91 (m, 2H), 2.95 (m, 2H), 3.28 (t, 2H), 3.58 (t, 2H), 3.85 (t, 2H), 6.78 (d, 1H), 6.88 (d, 1H), 7.85 (t, 1H), 7.96 (d, 1H), 8.45 (t, 1H), 8.78 (d, 1H), 9.17 (brs, 2H), 10.17 (brs, 1H), 11.78 (brs, 1H);
   Analysis: Found; C,47.69; H,6.08; N,7.79; S,10.88; Cl,12.84%,
   C₂₁H₂₇N₃O₃S₂.2HCl 1.5H₂O requires: C,47.27; H,6.05; N,7.88; S,12.02; Cl,13.29%.

## Claims

1. Compounds of formula I, 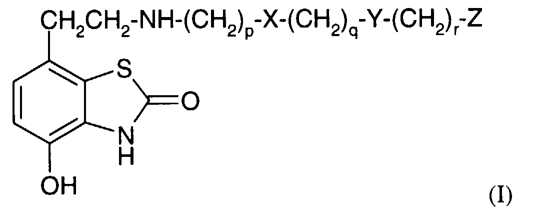 wherein X and Y independently represent -S(O)ₙ- or -O-,
n represents 0, 1 or 2,
p, q and r independently represent 2 or 3,
Z represents phenyl optionally substituted by halogen, hydroxyl, methoxy,nitro or amino, or represents a pyridyl or thienyl group,
and pharmaceutically-acceptable salts thereof.

2. A compound of formula I as defined in Claim 1 wherein Z represents phenyl.

3. A compound of formula I as defined in Claim 1 or Claim 2, wherein r represents 2.

4. A compound of formula I as defined in any one of the preceding claims, wherein p+q represents 5.

5. A compound of formula I as defined in any one of the preceding claims, wherein Y represents O.

6. A compound of formula I as defined in any one of the preceding claims, wherein X represents -S- or -SO₂-.

7. A compound of formula I which is
4-Hydroxy-7-[2-[3-[2-[2-phenylethoxy]ethylsulphonyl]propylamino]ethyl]-1,3-benzothiazol-2(3H)-one,
4-Hydroxy-7-[2-[2-[3-[2-phenylethoxy]propoxy]ethylamino]ethyl]-1,3-benzothiazol -2(3H)one,
4-Hydroxy-7-[2-[3-[2-[2-phenylethoxy]ethoxy]propylamino]ethyl]-1,3-benzothiazol -2(3H)one,
4-Hydroxy-7-[2-[2-[2-[2-phenylethoxy]ethoxy]ethylamino]ethyl]-1,3-benzothiazol -2(3H)-one,
4-Hydroxy-7-[2-[2-[3-[2-phenylethoxy]propylthio]ethylamino]ethyl]-1,3-benzothiazol -2(3H)-one,
4-Hydroxy-7-[2-[2-[3-[2-phenylethoxypropylsulphonyl]ethylamino]ethyl]-1,3-benzothiazol-2(3H)-one,
4-Hydroxy-7-[2-[3-[2-[2-phenylethoxy]ethylthio]propylamino]ethyl]-1,3-benzothiazol -2(3H)one,
4-Hydroxy-7-[2-[3-[2-[2-[-2-aminophenyl]ethoxy)ethylsulphonyl]propylamino]ethyl] -1,3-benzothiazol-2(3H)-one,
4-Hydroxy-7-[2- [3-[2-[2-[4-nitrophenyl]ethoxy]ethylsulphonyl]propylamino]-ethyl] -1,3-benzothiazol-2(3H)-one,
7-[2-[2-[3-[2-[4-Fluorophenyl]ethoxy]propylsulphonyl]ethylamino]ethyl]-4-hydroxy-1,3-benzothiazol-2(3H)-one,
4-Hydroxy-7-[2-[2-[3-[2-[2-thienyl]ethoxy]propylthio]ethylamino]ethyl]-1,3-benzothiazol-2(3H)one, or
4-Hydroxy-7-[2-[3-[2-[2-[2-pyridyl]ethoxy]ethylthio]propylamino]ethyl]-1,3-benzothiazol-2(3H)-one,
or a pharmaceutically acceptable salt of any one thereof.

8. A pharmaceutical composition comprising a compound of formula I, as defined in any one of the preceding Claims, or a pharmaceutically acceptable salt thereof, in association with a pharmaceutically acceptable diluent or carrier.

9. A method for the preparation of a compound of formula I, as defined in any one of Claims 1 to 7, or a pharmaceutically acceptable salt thereof, which comprises
a) alkylation of a compound of formula II, 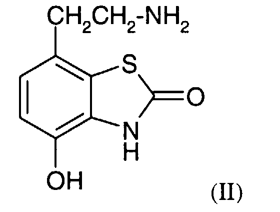 with an alkylating agent of formula III,
L-(CH₂)ₚ-X-(CH₂)_{q}-Y-(CH₂)ᵣ-Z (III)
in which p, q, r, X, Y and Z are as defined in Claim 1 and L represents a leaving group,
b) alkylation of a compound of formula II, as defined hereinabove, with a compound of formula IV,
O=CH-(CH₂)₍ₚ₋₁₎-X-(CH₂)_{q}-Y-(CH₂)ᵣ-Z (IV)
in which p, q, r, X, Y and Z are as defined in Claim 1, in the presence of a reducing agent,
c) selective reduction of a compound of formula V, 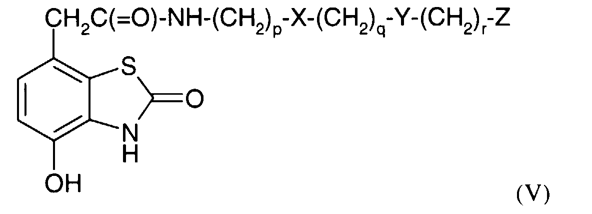 in which p, q, r, X, Y and Z are as defined in Claim 1,
d) selective reduction of a compound of formula Va, 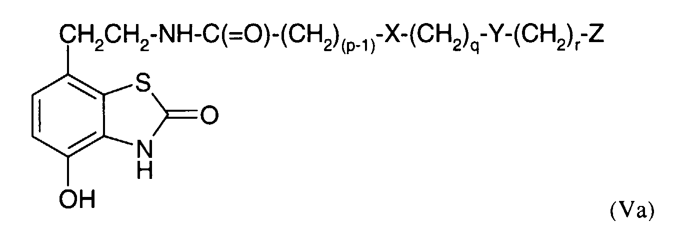 in which p, q, r, X, Y and Z are as defined in Claim 1,
e) removal of a protecting group from a corresponding protected compound of formula I in which one or more of the functional groups is protected,
and where desired or necessary converting a compound of formula I to a pharmaceutically-acceptable salt thereof, or vice versa.

10. Compounds of formula Va, 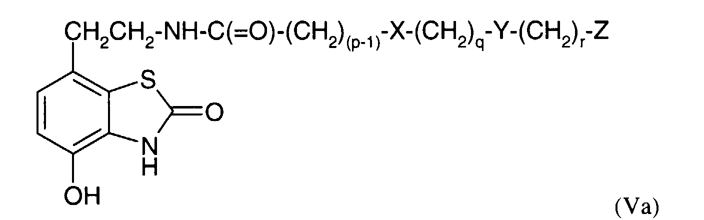 in which p, q, r, X, Y and Z are as defined in Claim 1.

## Patentansprüche

1. Verbindungen der Formel I 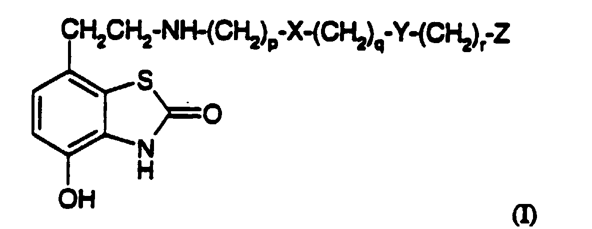 worin X und Y unabhängig -S(O)ₙ- oder -O- bedeuten,
n 0, 1 oder 2 bedeutet,
p, q und r unabhängig 2 oder 3 bedeuten,
Z gegebenenfalls durch Halogen, Hydroxy, Methoxy, Nitro oder Amino substituiertes Phenyl oder eine Pyridyl- oder Thienylgruppe bedeutet,
sowie deren pharmazeutisch unbedenkliche Salze.

2. Verbindung der Formel I nach Anspruch 1, worin Z Phenyl darstellt.

3. Verbindung der Formel I nach Anspruch 1 oder Anspruch 2, worin r 2 darstellt.

4. Verbindung der Formel I nach einem der vorhergehenden Ansprüche, worin p+q 5 darstellt.

5. Verbindung der Formel I nach einem der vorhergehenden Ansprüche, worin Y O darstellt.

6. Verbindung der Formel I nach einem der vorhergehenden Ansprüche, worin X -S- oder -SO₂- darstellt.

7. Verbindung der Formel I, wobei es sich um
4-Hydroxy-7-[2-[3-[2-[2-phenylethoxy]ethylsulphonyl]propylamino]ethyl]-1,3-benzothiazol-2(3H)-on,
4-Hydroxy-7-[2-[2-[3-[2-phenylethoxy]propoxy]ethylamino]ethyl]-1,3-benzothiazol-2(3H)-on,
4-Hydroxy-7-[2-[3-[2-[2-phenylethoxy]ethoxy]propylamino]ethyl]-1,3-benzothiazol-2(3H)-on,
4-Hydroxy-7-[2-[2-[2-[2-phenylethoxy]ethoxy]ethylamino]ethyl]-1,3-benzothiazol-2(3H)-on,
4-Hydroxy-7-[2-[2-[3-[2-phenylethoxy]propylthio]ethylamino]ethyl]-1,3-benzothiazol-2(3H)-on,
4-Hydroxy-7-[2-[2-[3-[2-phenylethoxy]propylsulphonyl]ethylamino]ethyl]-1,3-benzothiazol-2(3H)-on,
4-Hydroxy-7-[2-[3-[2-[2-phenylethoxy]ethylthio]propylamino]ethyl]-1,3-benzothiazol-2(3H)-on,
4-Hydroxy-7-[2-[3-[2-[2-[-2-aminophenyl]ethoxy]ethylsulphonyl]propylamino]ethyl]1,3-benzothiazol-2(3H)-on,
4-Hydroxy-7-[2-[3-[2-[2-[4-nitrophenyl]ethoxy]ethylsulphonyl]propylamino]-ethyl]-1,3-benzothiazol-2(3H)-on,
7-[2-[2-[3-[2-[4-Fluorphenyl]ethoxy]propylsulfonyl]ethylamino]ethyl]4-hydroxy-1,3-benzothiazol-2(3H)-on,
4-Hydroxy-7-[2-[2-[3-[2-[2-thienyl]ethoxy]propylthio]ethylamino]ethyl]1,3-benzothiazol-2(3H)-onoder
4-Hydroxy-7-[2-[3-[2-[2-[2-pyridyl]ethoxy]ethylthio]propylamino]ethyl]1,3-benzothiazol-2(3H)-on
oder ein pharmazeutisch unbedenkliches Salz von diesen handelt.

8. Pharmazeutische Zusammensetzung enthaltend eine Verbindung der Formel I nach einem der vorhergehenden Ansprüche oder eines ihrer pharmazeutisch unbedenklichen Salze gemeinsam mit einem pharmazeutisch unbedenklichen Streckmittel oder Trägerstoff.

9. Verfahren zur Herstellung einer Verbindung der Formel I nach einem der Ansprüche 1 bis 7 oder eines ihrer pharmazeutisch unbedenklichen Salze, dadurch gekennzeichnet, daß man
a) eine Verbindung der Formel II 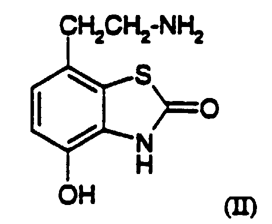 mit einem Alkylierungsmittel der Formel III,
L-(CH₂)ₚ-X-(CH₂)_{q}-Y-(CH₂)ᵣ-Z (III)
in welchem p, q, r, X, Y und Z wie in Anspruch 1 definiert sind und L eine Abgangsgruppe darstellt, alkyliert,
b) eine Verbindung der Formel II wie oben definiert mit einer Verbindung der Formel IV,
O=CH-(CH₂)₍ₚ₋₁₎-X-(CH₂)_{q}-Y-(CH₂)ᵣ-Z (IV)
in welcher p, q, r, X, Y und Z wie in Anspruch 1 definiert sind, in Gegenwart eines Reduktionsmittels alkyliert,
c) eine Verbindung der Formel V, 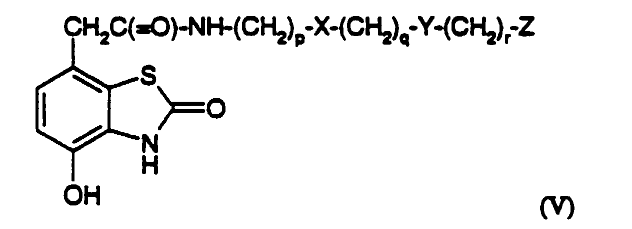 in welcher p, q, r, X, Y und Z wie in Anspruch 1 definiert sind,
selektiv reduziert,
d) eine Verbindung der Formel Va, 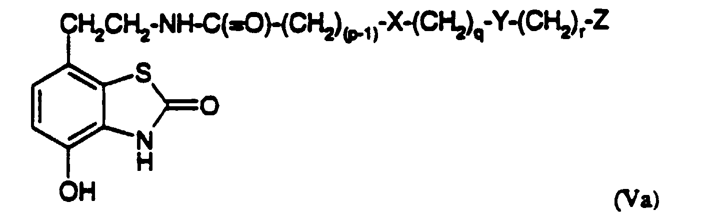 in welcher p, q, r, X, Y und Z wie in Anspruch 1 definiert sind,
selektiv reduziert,
e) von einer entsprechenden geschützten Verbindung der Formel I, in welcher eine oder mehrere funktionelle Gruppen geschützt ist/sind, eine Schutzgruppe entfernt,
und, falls erwünscht oder erforderlich, eine Verbindung der Formel I in eines ihrer pharmazeutisch unbedenklichen Salze umwandelt oder umgekehrt.

10. Verbindungen der Formel Va, 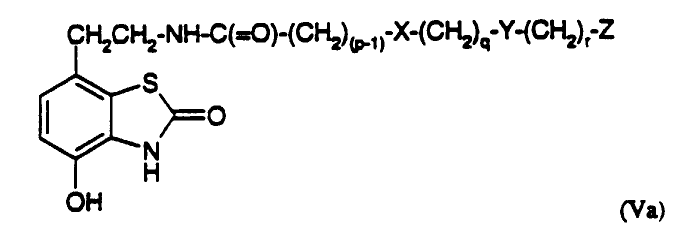 in welcher p, q, r, X, Y und Z wie in Anspruch 1 definiert sind.

## Revendications

1. Composés de formule I : 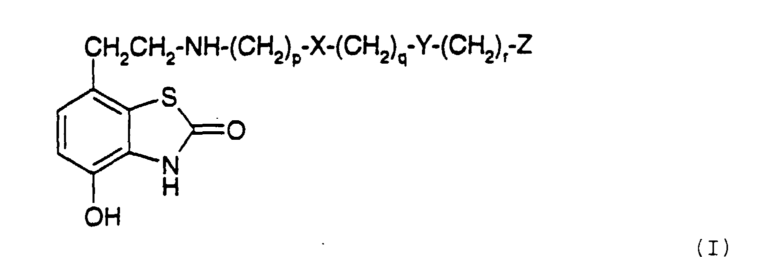 dans laquelle X et Y représentent indépendamment -S(O)ₙ- ou -O-,
n représente 0, 1 ou 2,
p, q et r représentent indépendamment 2 ou 3,
Z représente un groupe phényle éventuellement substitué par un halogène ou un groupe hydroxyle, méthoxy, nitro ou amino, ou représente un groupe pyridyle ou thiényle, et leurs sels pharmaceutiquement acceptables.

2. Composé de formule I selon la revendication 1, dans lequel Z représente un groupe phényle.

3. Composé de formule I selon la revendication 1 ou 2, dans lequel r représente 2.

4. Composé de formule I selon l'une quelconque des revendications précédentes, dans lequel la somme p + q représente 5.

5. Composé de formule I selon l'une quelconque des revendications précédentes, dans lequel Y représente O.

6. Composé de formule I selon l'une quelconque des revendications précédentes, dans lequel X représente -S- ou -SO₂-.

7. Composé de formule I qui est l'un des suivants :
4-hydroxy-7-[2-[3-[2-[2-phényléthoxy]éthylsulfonyl] propylamino]éthyl]-1,3-benzothiazol-2(3H)-one,
4-hydroxy-7-[2-[2-[3-[2-phényléthoxy]propoxy] éthylamino]éthyl]-1,3-benzothiazol-2(3H)-one,
4-hydroxy-7-[2-[3-[2-[2-phényléthoxy]éthoxy]propylamino] éthyl]-1,3-benzothiazol-2(3H)-one,
4-hydroxy-7-[2-[2-[2-[2-phényléthoxy]éthoxy]éthylamino] éthyl]-1,3-benzothiazol-2(3H)-one,
4-hydroxy-7-[2-[2-[3-[2-phényléthoxy]propylthio] éthylamino]éthyl]-1,3-benzothiazol-2(3H)-one,
4-hydroxy-7-[2-[2-[3-[2-phényléthoxy]propylsulfonyl] éthylamino]éthyl]-1,3-benzothiazol-2(3H)-one,
4-hydroxy-7-[2-[3-[2-[2-phényléthoxy]éthylthio] propylamino]éthyl]-1,3-benzothiazol-2(3H)-one,
4-hydroxy-7-[2-[3-[2-[2-[2-aminophényl]éthoxy] éthylsulfonyl]propylamino]éthyl]-1,3-benzothiazol-2(3H)-one,
4-hydroxy-7-[2-[3-[2-[2-[4-nitrophényl]éthoxy] éthylsulfonyl]propylamino]-éthyl]-1,3-benzothiazol-2(3H)-one,
7-[2-[2-[3-[2-[4-fluorophényl]éthoxy]propylsulfonyl] éthylamino]éthyl]-4-hydroxy-1,3-benzothiazol-2(3H)-one,
4-hydroxy-7-[2-[2-[3-[2-[2-thiényl]éthoxy]propylthio] éthylamino]éthyl]-1,3-benzothiazol-2(3H)-one ou
4-hydroxy-7-[2-[3-[2-[2-[2-pyridyl]éthoxy]éthylthio] propylamino]éthyl]-1,3-benzothiazol-2(3H)-one,
ou un sel pharmaceutiquement acceptable de l'une quelconque de ces substances.

8. Composition pharmaceutique comprenant un composé de formule I selon l'une quelconque des revendications précédentes ou un sel pharmaceutiquement acceptable de celui-ci en association avec un diluant ou un véhicule pharmaceutiquement acceptable.

9. Procédé de préparation d'un composé de formule I selon l'une quelconque des revendications 1 à 7 ou d'un sel pharmaceutiquement acceptable de celui-ci, qui comprend :
a) l'alkylation d'un composé de formule II: 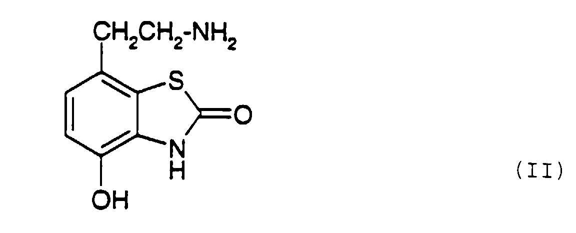 avec un agent d'alkylation de formule III :
L-(CH₂)ₚ-X-(CH₂)_{q}-Y-(CH₂)ᵣ-Z (III)
dans laquelle p, q, r, X, Y et Z sont tels que définis dans la revendication 1 et L représente un groupe quittant,
b) l'alkylation d'un composé de formule II tel que défini ci-dessus avec un composé de formule IV :
O=CH-(CH₂)_{(P-1)}-X-(CH₂)_{q}-Y-(CH₂)ᵣ-Z (IV)
dans laquelle, p, q, r, X, Y et Z sont tels que définis dans la revendication 1, en présence d'un agent réducteur,
c) la réduction sélective d'un composé de formule V : 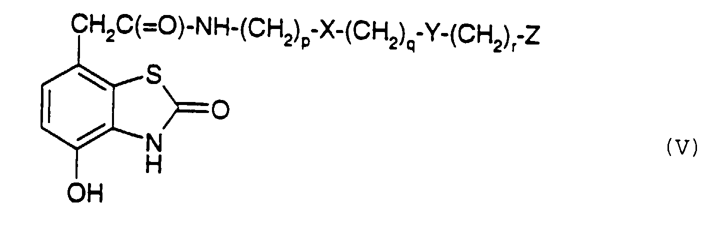 dans laquelle p, q, r, X, Y, et Z sont tels que définis dans la revendication 1,
d) la réduction sélective d'un composé de formule Va : 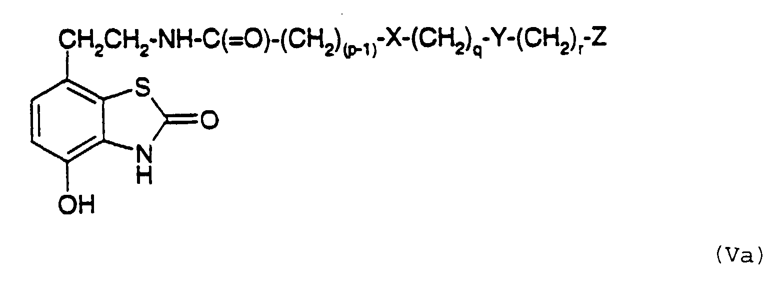 dans laquelle p, q, r, X, Y et Z sont tels que définis dans la revendication 1,
e) l'élimination d'un groupe protecteur d'un composé protégé correspondant de formule I, dans lequel un ou plusieurs des groupes fonctionnels sont protégés, et, lorsqu'on le souhaite ou lorsque cela s'avère nécessaire, la conversion d'un composé de formule I en un sel pharmaceutiquement acceptable de celui-ci ou vice versa.

10. Composés de formule Va : 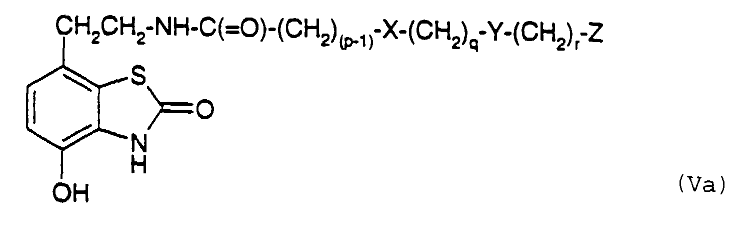 dans laquelle p, q, r, X, Y et Z sont tels que définis dans la revendication 1.
